(19) Europäisches Patentamt European Patent Office Office européen des brevets

(11) **EP 4 542 566 A1**

(12) **EUROPEAN PATENT APPLICATION**

(43) Date of publication:
23.04.2025 Bulletin 2025/17

(21) Application number: 23208229.7

(22) Date of filing: 07.11.2023

(51) International Patent Classification (IPC):
**G16H 40/20** (2018.01)    **G06Q 10/06** (2023.01)

(52) Cooperative Patent Classification (CPC):
**G16H 40/20; G06Q 10/06; G06Q 10/06312;
G06Q 10/06315; G06Q 10/0637**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.10.2023 US 202363591771 P**

(71) Applicant: **Koninklijke Philips N.V.
5656 AG Eindhoven (NL)**

(72) Inventors:
- **MENDOZA, Jose Luis Diaz**
  **Eindhoven (NL)**
- **MUIJLWIJK, Heleen**
  **Eindhoven (NL)**
- **VAN EE, Raymond**
  **5656AG Eindhoven (NL)**
- **CHAKRABARTI, Biswaroop**
  **Eindhoven (NL)**

(74) Representative: **Philips Intellectual Property &
Standards
High Tech Campus 52
5656 AG Eindhoven (NL)**

(54) **PLANNING UPDATES FOR AN INTERDEPENDENT FLEET OF MEDICAL DEVICES**

(57) In planning an upcoming update or upgrade of a fleet of medical devices, historical workload information are received for medical devices of the fleet. Cost preferences are received for respective costs of the upcoming update or upgrade. Values for the respective costs of the upcoming update or upgrade of the fleet are estimated for different scenarios for performing the upcoming update or upgrade. One or more proposed scenarios are selected for performing the upcoming update or upgrade of the fleet of medical devices based on comparisons of the estimated values for the respective costs with the cost preferences for the respective costs received via the UI. Information is displayed about the selected one or more scenarios for performing the upcoming update or upgrade of the fleet of medical devices including at least indications of the estimated values for the respective costs.

Fig. 1

EP 4 542 566 A1

**Description**

FIELD OF THE INVENTION

The following generally relates to the medical device update or upgrade arts, medical imaging device update or upgrade arts, medical device update or upgrade planning arts, and related arts.

BACKGROUND OF THE INVENTION

Medical devices such as computed tomography (CT) scanners, magnetic resonance imaging (MRI) scanners, positron emission tomography (PET) scanners, image-guided therapy (IGT) devices, and other medical imaging devices are mechanically complex and highly computerized. As just one example, an MRI scanner may include thousands of systems, subsystems, and individual components, with automated operation of the patient support bed, magnetic field gradient coils, radio frequency (RF) coils, and other hardware, along with software for reconstructing and displaying acquired MRI images. The maintenance of such complex medical devices is often outsourced to the device vendor since hospitals typically do not have the requisite expertise. In addition to maintenance, the vendor party may occasionally offer software or hardware updates or upgrades to improve the capabilities of the medical device, or for another reason such as compliance with new or revised governmental regulations. Scheduling such an update or upgrade can be challenging, as an MRI or other complex medical device commonly handles a heavy workload. The update or upgrade should minimally interfere with that workload.

Updates and upgrades of medical devices inevitably result in downtime, and such downtime has costs in terms of lost revenue from radiology examinations performed with the medical devices, patient rescheduling costs, and/or so forth. In order to plan updates and upgrades, hospitals need to deal with alternative allocation of patients and staff, often resulting in capacity issues. When updating across the whole fleet of medical devices, this alternative allocation is becoming an even bigger challenge. For hospitals it is not straightforward how to deal with this, and there is an unmet need for systematic adaptive methods for optimization.

The following discloses certain improvements to overcome these problems and others.

SUMMARY OF THE INVENTION

In one aspect, a system for planning an upcoming update or upgrade of a fleet of medical devices, the system including a hardware processor, a display device, and a non-transitory computer readable medium. The non-transitory computer readable medium stores instructions readable and executable by the hardware processor to: receive historical workload information for medical devices of a fleet of medical devices, the fleet including at least two medical devices; receive, via a user interface (UI) presented on the display device, cost preferences for respective costs of the upcoming update or upgrade of the fleet of medical devices, the respective costs including at least a patient delay cost, a patient rescheduling cost, and a staff overtime cost; estimate values for the respective costs for different scenarios for performing the upcoming update or upgrade of the fleet of medical devices, each scenario specifying a schedule for the upcoming update or upgrade of the fleet of medical devices and resources allocated for the upcoming update or upgrade of the fleet of medical devices, wherein the values estimated for the costs for each scenario are metrics of the impact of performing the upcoming update or upgrade in accordance with the scenario on efficiency of operation of the fleet of medical devices during the upcoming update or upgrade; select one or more proposed scenarios for performing the upcoming update or upgrade of the fleet of medical devices based on comparisons of the estimated values with the cost preferences for the respective costs received via the UI; and display, on the display device, information about the selected one or more scenarios for performing the upcoming update or upgrade of the fleet of medical devices including at least indications of the estimated values for the respective costs.

In another aspect, in a system as set forth in the immediately preceding paragraph, the instructions are further readable and executable by the hardware processor to: receive, via the UI, a selection of a user-selected scenario from the selected one or more scenarios; and automatically perform at least a portion of the upcoming update or upgrade of the fleet of medical devices in accordance with the user-selected scenario.

In another aspect, a method is disclosed for planning an upcoming update or upgrade of a fleet of medical devices. The method includes: receiving historical workload information for medical devices of the fleet of medical devices; receiving, via a user interface (UI), cost preferences for respective costs of the upcoming update or upgrade of the fleet of medical devices; estimating values for the respective costs of the upcoming update or upgrade of the fleet of medical devices for different scenarios for performing the upcoming update or upgrade of the fleet of medical devices, each scenario specifying at least a schedule for the upcoming update or upgrade of the fleet of medical devices and whether pairs of medical devices of the fleet are to be updated or upgraded simultaneously or consecutively; selecting one or more proposed scenarios for performing the upcoming update or upgrade of the fleet of medical devices based on comparisons of the estimated values for the respective costs with the cost preferences for the respective costs received via the UI; and outputting, on the UI, information about the selected one or more scenarios for performing the upcoming update or upgrade of the fleet of medical devices including at least indications of the estimated values for the respective costs.

In another aspect, the method of the immediately preceding paragraph further includes: receiving, via the UI, a selection of a user-selected scenario from the selected one or more scenarios; and automatically performing at least a portion of the upcoming update or upgrade of the fleet of medical devices in accordance with the user-selected scenario.

In another aspect, a non-transitory computer readable medium stores instructions readable and executable by at least one hardware processor to: receive historical workload information for medical devices of a fleet of medical devices, the fleet including at least two medical devices; receive information on an upcoming update or upgrade of the fleet of medical devices; receive information on resources available for performing the upcoming update or upgrade of the fleet of medical devices; receive, via a user interface (UI), cost preferences for respective costs of the upcoming update or upgrade of the fleet of medical devices; simulate different scenarios performing the upcoming update or upgrade of the fleet of medical devices, each scenario specifying a schedule for the upcoming update or upgrade of the fleet of medical devices and resources allocated for the upcoming update or upgrade of the fleet of medical devices, the simulation of each scenario utilizing the historical workload information for the medical devices of the fleet of medical devices and outputting estimated values for the respective costs of the upcoming update or upgrade of the fleet of medical devices; select one or more proposed scenarios for performing the upcoming update or upgrade of the fleet of medical devices from the simulated different scenarios based on comparisons of the estimated values for the respective costs output by the simulations of the different scenarios with the cost preferences for the respective costs received via the UI; and output, on the UI, information about the selected one or more scenarios for performing the upcoming update or upgrade of the fleet of medical devices including at least indications of the estimated values for the respective costs.

One advantage resides in planning updates or upgrades for a fleet of medical devices based on an interdependency of the medical devices.

Another advantage resides in planning updates or upgrades for a fleet of medical devices based on resources and schedules at a medical facility where the medical devices are located.

Another advantage resides in providing a user interface with slider bars allowing a user to optimize a plan for scheduling updates or upgrades for a fleet of medical devices.

Another advantage resides in providing an intuitive user interface to ease the task of the fleet manager or updates planner, considering different scenarios where the update of one device can affect the normal operation of other devices.

Another advantage resides in providing a model to consider relevant costs of downtime of a device due to an upcoming update or upgrade, involving relevant persons in the optimal solution, such as patients, staff member(s) operating such devices or service provider's availability.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

Fig. 1 diagrammatically illustrates an illustrative system for determining an upgrade or update session for servicing a medical device in accordance with the present disclosure.
Fig. 2 shows exemplary flow chart operations of the system of Fig. 1.
Fig. 3 shows another embodiment of the system of Fig. 1.
Fig. 4 presents a Venn diagram of a decision made by the system of Fig. 1.

DETAILED DESCRIPTION OF EMBODIMENTS

When updating hardware and/or software of a fleet of medical devices, there will typically be downtime of the medical devices, with consequent delay or rescheduling of patients, lost revenue if medical examinations are outsourced during the downtime, staff overtime costs, and/or so forth. Customers (e.g., hospitals or other medical institutions) can value these factors differently: some customers may prefer to pay staff overtime to reduce patient delays or rescheduling, while other customers may prefer to close the relevant medical department or laboratory entirely to minimize staff costs at the expense of patient delays or rescheduling. Typically, customer preferences are accommodated by way of consultations between the customer and the servicing entity's customer representative. This ad hoc approach can lead to customer dissatisfaction and implementing a nonoptimal upgrade pathway.

The following discloses a system for planning a fleet update in an automated fashion. The system includes a user interface (typically customer-facing) and a backend optimization module that employs a disclosed total cost estimation for a given upgrade scenario. In use, the customer uses slider bars or other user input dialogs of the user interface to indicate preferences for the various costs, which can include patient delay costs, patient rescheduling costs, and staff overtime costs. The backend optimization also takes into account whether the updates are performed simultaneously or consecutively in computing the overall cost. For example, if a fleet of medical imaging devices including two magnetic resonance imaging (MRI) scanners is being updated, there may be benefits to updating the MRI scanners consecutively as this can keep one MRI scanner available for use throughout the upgrade. Conversely, there may be benefits to updating the MRI scanners simultaneously, such as allowing the MRI department to be shut down entirely during the update thus saving staff costs. In general, it is desired to not shut down the entire fleet of medical devices, but rather to continue medical operations as efficiently as possible during the upgrade, using at least a subset of the medical devices of the fleet while the upgrade is in-progress. Hence, the cost of an upgrade scenario can be a quantification of the impact of the upgrade scenario on processing efficiency of operation of the fleet of medical devices as the upgrade is being performed (e.g., in the background).

Typically, a fleet upgrade may involve up to a dozen or more medical devices being updated simultaneously. In assessing the costs of simultaneous versus consecutive updating, this can be done for every possible grouping of the (e.g., 12) medical devices, leading to a computationally challenging task. The following provides an optimization algorithm that provides for doing this.

A further aspect of the illustrative embodiment is to use artificial intelligence (AI) to learn customer preferences for various cohorts or even individual customers. This enables the sliders or other user input dialogs to be initially set to personalized default values which can expedite the fleet update planning process.

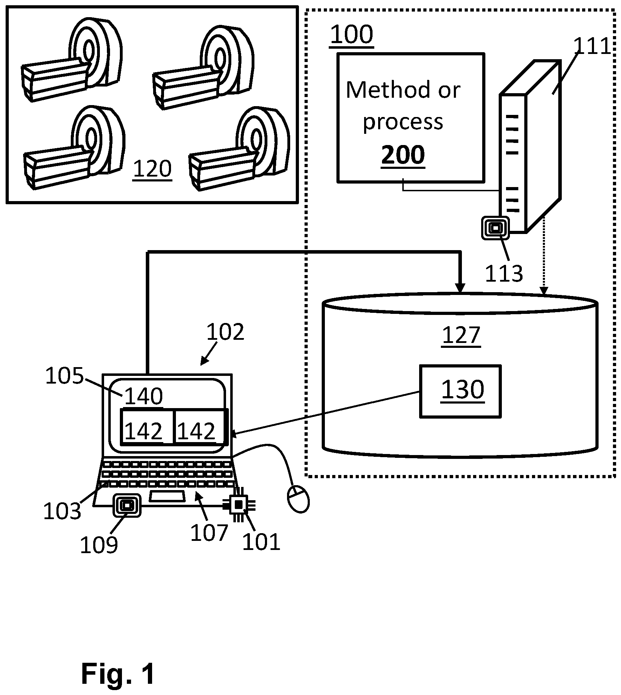

With reference to Fig. 1, an illustrative scheduler system or apparatus **100** implemented on a server computer **111** or the like provides for scheduling an update or upgrade or other maintenance procedure of a fleet of devices **120** (e.g., a medical imaging device - also referred to as a medical device, an imaging device, imaging scanner, and variants thereof) is diagrammatically shown. While four medical devices are shown by way of illustrations in Fig. 1, it will be understood that the fleet of medical devices **120** can include one, two, three, illustrative four, five, six, seven, eight, nine, ten, eleven, twelve, or more medical devices **120**, as further examples. By way of some non-limiting illustrative examples, the medical imaging device **120** to be scheduled to undergo service may be a magnetic resonance imaging (MRI) scanner, a computed tomography (CT) scanner, a positron emission tomography (PET) scanner, a gamma camera for performing single photon emission computed tomography (SPECT), an interventional radiology (IR) device, or so forth. (More generally, embodiments of the disclosed scheduler can be applied in conjunction with scheduling of an update or upgrade of any type of complex computerized device, e.g., the approach could be applied to schedule an update or upgrade of a commercial airliner, radiation therapy device, or so forth). As shown in Fig. 1, the scheduler **100** includes, or is accessible by, a customer device **102** that may for example be a workstation or electronic processing device used by the customer (e.g., a fleet manager, planner, or other representative of the hospital or other medical institution who is the customer receiving the upgrade). The customer device **102** is operated by an employee or other agent of the customer, and may for example be a desktop computer or a portable device such as a notebook computer. As another nonlimiting illustrative example, the customer device **102** may be a mobile device such as a cellular telephone (cellphone) or tablet computer.

The customer device **102** includes a display **105**, and at least one user input device **103** such a mouse, keyboard, or touchscreen. The customer device **102** further includes an electronic processer **101** and non-transitory storage medium **107** (internal components which are diagrammatically indicated in Fig. 1). The non-transitory storage medium **107** stores instructions which are readable and executable by the electronic processor **101** for interfacing the customer with the update or upgrade scheduler system **100**. The customer device **102** also includes a communication interface **109** to communicate with the server or processing device **111**, which implements the computational aspects of the update or upgrade scheduler system **100**. Such communication interfaces **109** include, for example, a wired and/or wireless Ethernet interface (e.g., in the case in which the customer device **102** is a workstation); or in the case in which the customer device **102** is a notebook computer, or other portable device the interface **109** may be a wireless Wi-Fi or 4G/5G interface or the like for connection to the Internet and/or an intranet. In some embodiments, the customer device **102** interfaces with the scheduler system **100** by way of a webpage or the like that is accessed via the Internet and presented on a web browser running on the customer device **102**. In other embodiments, the customer device **102** interfaces with the scheduler system **100** by way of an application program running on the customer device **102** - in this case, the customer may initially download the application program from the server **111**. Some aspects of the scheduler system **100** may also be implemented by cloud processing or other remote processing (that is, the server computer **111** may be embodied as a cloud-based computing resource comprising a plurality of interconnected servers). In illustrative Fig. 1, the update or upgrade scheduler **100** is implemented and/or owned by the imaging device vendor or leased by the vendor from a cloud computing service provider.

The vendor device **102** is programmed to interact with the update or upgrade scheduler **100** to provide inputs to set up for an upcoming update or upgrade session for the medical device **120**. To schedule the update or upgrade session, the scheduler **100** analyzes the received one or more inputs, optionally along with information about the customer, to determine a scenario for performing the upcoming update or upgrade of the fleet **120** of medical devices (for example, including a proposed time for performing the upcoming update or upgrade session, whether medical devices should be updated or upgraded simultaneously or consecutively, and/or so forth). This proposed scenario is output on a user interface (UI) **140** of the vendor device **102**.

The server **111** implementing the scheduler **100** is equipped with non-transitory storage medium **127** (internal components which are diagrammatically indicated in Fig. 1). While a single server computer is shown, it will be appreciated that the backend **110** may more generally be implemented on a single server computer, or a server cluster, or a cloud computing resource comprising ad hoc-interconnected server computers, or so forth.

With continuing reference to Fig. 1, the non-transitory computer readable medium **127** stores a model **130** configured to analyze cost preferences for respective costs of the upcoming update or upgrade of the fleet of medical devices **12** to estimate the values for the respective costs for the different scenarios for the medical device **120**. For example, the customer agent provides inputs via the customer device **102** related to customer preferences for the upcoming update or upgrade session, which are received and analyzed by the model **130**. In some embodiments, the model **130** comprises a Bayesian model. The model **130** includes one or more conditions that limit when the update or upgrade session can be performed. These conditions may be different for different customers and may be parameters of the Bayesian or other model **130**. While a Bayesian model is employed as an illustrative model **130**, other types of models can be used for analyzing the received inputs to determine a proposed time for performing the upcoming update or upgrade session, such as Monte Carlo modeling, a suitably trained artificial neural network (ANN), or so forth.

The non-transitory storage medium **127** further stores instructions executable by the electronic processor **113** of the backend server **111** to perform an update or upgrade scheduling method **200** for planning an upcoming update or upgrade of a fleet of medical devices **120**. In other embodiments, maintenance of hardware, or combined hardware maintenance software updating, sessions may be similarly scheduled.

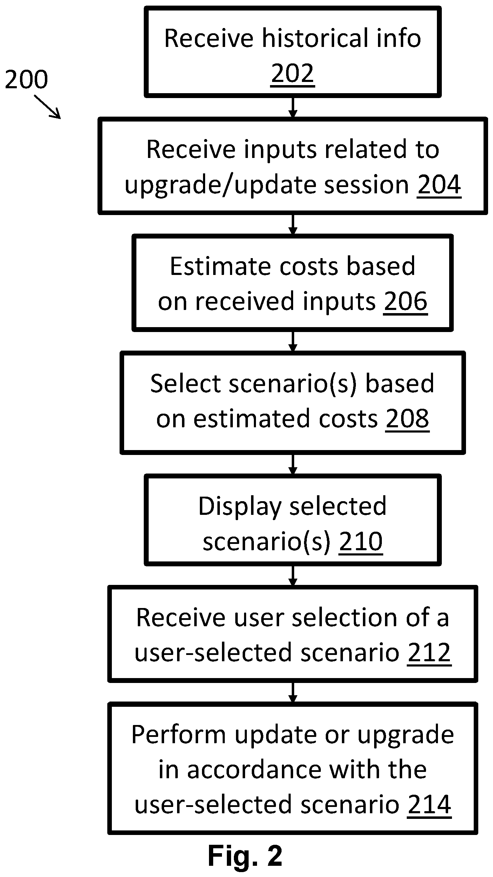

With continuing reference to Fig. 1 and further reference to Fig. 2, an illustrative embodiment of the update or upgrade scheduling method **200** executable by the electronic processor **113** is diagrammatically shown as a flowchart. In some examples, the scheduling method **200** may be performed at least in part by cloud processing.

To begin the method **200**, at an operation **202**, historical workload information for medical devices of a fleet of medical devices **120** are received at the server **111**, for example from the non-transitory computer readable medium **127**. In some embodiments, information on resources available for performing the upcoming update or upgrade of the fleet of medical devices **120** is also received, for example from the non-transitory computer readable medium **127**.

At an operation **204**, the GUI **140** is presented on the display device **150** of the customer device **102**. The GUI **140** may, for example, be presented as a webpage displayed in a web browser running on the customer device **102**, or may be presented by an application program running on the customer device **102**. Via the GUI **140**, the user provides inputs via the user input device(s) **103** indicative of cost preferences for respective costs of the upcoming update or upgrade of the fleet of medical devices **120**. The respective costs can include for example, at least a patient delay cost, a patient rescheduling cost, a staff overtime cost, and so forth. More generally, the costs are metrics of the impact of the upgrade scenario on processing efficiency of operation of the fleet of medical devices as the update or upgrade is being performed. In this view, the upgrade constitutes a background process that is performed while at least a subset of the medical devices of the fleet continue to be utilized to provide medical care, and the costs are metrics of the impact of the upgrade background process on the efficiency of the fleet of medical devices as they continue operate to provide medical services during the upgrade. To provide the inputs, the GUI **140** displays one or more slider bars **142** for each respective cost of the upcoming update or upgrade of the fleet of medical devices **120**, and the inputs setting the cost preferences are received as adjustments of the slider bars **120**. While slider bars **142** are illustrated, other types of user dialogs are also contemplated, such as providing numerical "minimum" and "maximum" value inputs for various costs.

In some embodiments, default cost preferences can be determined based on cost preferences received via the GUI **140** for previous updates or upgrades of the fleet of medical devices **120** and/or similar fleets of medical devices **120**. To do so, the GUI **140** can be initialized with the default cost preferences before the user adjusts the slider bars **142**.

At an operation **206**, values for the respective costs for different scenarios are estimated for performing the upcoming update or upgrade of the fleet of medical devices **120**. Each scenario can specify a schedule for the upcoming update or upgrade of the fleet of medical devices **120** and resources allocated for the upcoming update or upgrade of the fleet of medical devices **120**. Each scenario further includes one or more updates for the fleet of medical devices **120**, patient care workflows, staff schedules, field service engineer schedules, and so forth. The different scenarios differ at least in whether the upcoming update or upgrade is performed simultaneously or consecutively for a pair of medical devices of the fleet of medical devices **120**. To estimate the values for the respective costs for the different scenarios, the model **130** is configured to analyze the cost preferences for respective costs of the upcoming update or upgrade of the fleet of medical devices **120**. The values estimated for the costs for each scenario in the operation **206** are metrics of the impact of performing the upcoming update or upgrade in accordance with the scenario on efficiency of operation of the fleet of medical devices during the upcoming update or upgrade.

At an operation **208**, one or more proposed scenarios for performing the upcoming update or upgrade of the fleet of medical devices **120** are selected based on comparisons of the estimated values with the cost preferences for the respective costs received via the UI **140**.

At an operation **210**, information about the selected one or more scenarios for performing the upcoming update or upgrade of the fleet of medical devices are displayed on the display device **105**. The displayed information can include, for example, indications of the estimated values for the respective costs.

At an operation **212**, a user selects, via the UI **140**, a user-selected scenario from the one or more scenarios for performing the upcoming update or upgrade of the fleet of medical devices that were selected in the operation **208**. The user suitably selects the user-selected scenario based on the information about the one or more (proposed) scenarios displayed in the operation **210**. The upcoming update or upgrade will then be performed in accordance with the user-selected scenario.

In some embodiments, the update or upgrade scheduling method **200** may be extended to include automated performance of at least a portion of the update or upgrade in accordance with the user-selected scenario selected at the operation **212**. To this end, in an optional further operation **214** at least a portion of the update or upgrade is automatically performed in accordance with the user-selected scenario. For example, if a portion or the entirety of the update or upgrade is a software update, then this can be automatically implemented in the operation **214** by pushing the software update from the server **111** to the appropriate medical devices **120**. In another example, if the medical devices **120** are computed tomography (CT) scanners or positron emission tomography (PET) scanners or the like which employ radiation detectors, and a portion or the entirety of the update or upgrade is a calibration of the radiation detectors, then the operation **214** may entail controlling the CT or PET scanner(s) to acquire calibration data of an imaging phantom, a point radiation source, or an empty scanner (i.e., an air scan or air calibration), and processing the calibration data to generate calibration scaling factors or the like for scaling values output by the detector elements of the radiation detectors. These are merely nonlimiting illustrative examples.

In other embodiments, the update or upgrade may be performed manually, for example if the update or upgrade entails manually performed replacement or addition of physical hardware of the medical device(s).

EXAMPLES

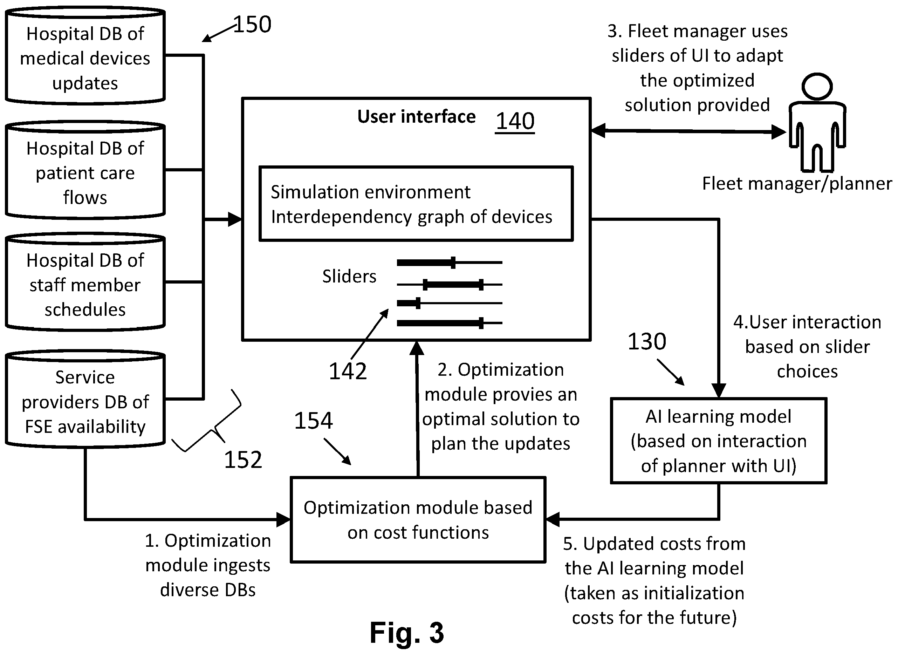

Fig. 3 shows another embodiment of the scheduler system **100**. As shown in Fig. 3, the system **100** can include one or more databases including a first database **150** operated by a hospital or medical institution, and stores activity and historical data of medical devices **120** which belong to the fleet, staff members who operate the devices availably, patients care flows which make use of the medical device, activity of medical devices from third party providers, and so forth. At least one second database **152** is operated by a service provider and stores data related to availability of field service engineers, updates / upgrades needed for medical devices supplied.

The server **111** is programmed to implement an optimization module **154** storing a mathematical or statistical model which computes the optimal solution to allocate updates or upgrades for the selected devices **120**. The optimization module **156** provides initialization costs to calculate the solution based on historical data.

The model **130** can comprise an artificial intelligence (AI) interaction learning model which learns the user (i.e., fleet manager) behavior of tuning the UI sliders **142** to adapt the recommendation from the optimization model or solver **154**. While learning the behavior over time some user behavior patterns which are not obvious can be captured.

The user interface **140** can display dashboards or graphs, including for example, dashboards of update or upgrade tasks to be approved to get executed, an interdependency graph of devices which interact most often together, an interdependency graph of staff members which interact mostly with the devices, an interdependency graph of the patients care flows or medical procedures which make use of certain devices, bottlenecks in the scheduling flow, alerts of malfunction of components of medical devices, the sliders **142**(or buttons) which the user can tune in to adapt the recommended solution to his / her needs, a simulation environment where the staff member can simulate different scheduling scenarios to assess the overall cost implied in each scenario, and so forth.

In some embodiments, the optimization module **154** is configured to compute an optimal scheduling solution. The cost functions can be used by the optimization module **154** to come up with the optimal solution. First, the explanation is focused on the costs involved to update a single device X and later on, the costs involved considering the interdependency of devices $X_1$ and $X_2$. For example, in a cost function for updating device X at time t, there is a cost function $C=fx(t)$ that describes what the costs are of updating (i.e., downtime) device X at time t. This cost function $fx(t)$ is different for different scenarios S, resulting in various cost functions $fx,s(t)$. In one example scenario, one can make the decision to shift or extend working hours of staff, shifting patients that were planned on devices-to-be-updated to similar devices after normal hours, to avoid or limit the patient waiting time and keep normal patient throughput. This can potentially affect, for example, a staff member $M_1$ will be asked to work during the evening instead of the day at costs $C_{M1}$, a staff member $M_2$ will be asked to work during the evening instead of the day at costs $C_{M2}$, and so forth.

In another example scenario, one can make the decision to delay certain patients, which were planned on the devices-to-be-updated, to a later point in time, with the cost of risking that patients' symptoms will increase, or resulting in capacity issues later. This affects potentially, for example, a first patient $P_1$ will be delayed to moment $t_{P_1}'$ with the cost of $C_{P_1}$, a second patient $P_2$ will be delayed to moment $t_{P_2}'$ with the cost of $C_{P_2}$, and so forth. This can potentially be extended with other variables, such as service provider availability, accessories with devices that become idle as well when device is down, an availability of the system (in terms of patient load, as it is beneficial to schedule updates when the worklist is short or empty), and so forth.

Thus, the total cost of updating device X under scenario S on moment t is defined by $\Sigma_{M,P} f_{X,S}(t)$, that is the summed costs of all the stakeholders (M, P, and perhaps more) involved.

In deciding upon or creating a certain scenario, there must be certain constraints in place. For example, the number of operators is related to the number of active devices (to make sure they are all 'operational'), and there might also be boundaries to the length of the shifts or reasonable timeslots to ask patients to come to the hospital.

In addition, costs of not updating device X can be considered. For example, by using predictive maintenance models we can estimate the probability that the device or components of the device will break down when not updated, which is likely to increase over time (similar to a depreciation cost). In another example, when new features are released and hospitals decide to not update and keep working with the old version, a cost element that represents inefficiency can be included to compare the use of the new versus the old version.

When one needs to decide when to update a device (i.e., set t) and how to deal with the scheduling (i.e., set S), one wants to choose that t and S that minimizes the total costs. Thus, a cost function to do so can include

$$\min_{S,t} \Sigma_{P,M} f_{X,S}(t)$$

.

For multiple devices, that is $X_1$, $X_2$, $X_3$, etc., the optimization module **154** can decide to update all or some devices together, in parallel, or after each other, consecutive. For example, the optimization module **154** can decide which subset of the fleet, $x \in X$, we update together at time t. The cost function of one device $X_1$ changes depending on whether another device $X_2$ should be updated. That is, the cost of updating one device depends on whether patients and staff can be allocated to other similar devices that are not down. This implies that the decisions to update multiple devices are independent, and the cost functions are not additive, resulting in a cost function of total $costs \neq \Sigma_{P,M} f_{X_1,S}(t') + \Sigma_{P,M} f_{X_2,S}(t')$. Table 1 shows a summary of considerations in deciding whether to update two devices **120** together or separately.

Table 1

|  | Updating device $X_1$ | Not updating device $X_1$ |
|---|---|---|
| Updating device $X_2$ | Updating both devices together can have **advantages:** total costs $< \Sigma_{P,M} f_{X_1,S}(t') + \Sigma_{P,M} f_{X_2,S}(t')$, for example in the case where you have 2 operators, and 1 secretary. When 1 operator is still working, the secretary also needs to be on duty, whereas when both are not operating, the whole 'department' can be down for some time, saving costs of, e.g., the secretary.<br><br>Updating both devices together can also have **drawbacks:** total costs $> \Sigma_{P,M} f_{X_1,S}(t') + \Sigma_{P,M} f_{X_2,S}(t')$, for example when you want to allocate patients to alternative devices, to maintain the patient throughput as much as possible. | For the benefits: see the drawbacks of updating them together in the top left cell. |
| Not updating device $X_2$ | For the benefits: see the drawbacks of updating them together in the top left cell. |  |

Ultimately the impact of updating multiple devices together is a combination of advantages and drawbacks, that all should be taken into consideration. Then, when the total costs of updating together are lower than the additive costs of updating them separately (total $costs < \Sigma_{P,M} f_{X_1,S}(t') + \Sigma_{P,M} f_{X_2,S}(t')$, one should update them together, if not, one should update them separately.

However, the above reasoning all implies to a given S and t. Of course, one should decide upon S and t first. This can be done given the total cost function, including the dependencies (i.e., knowing the cost function of updating $X_1$ when $X_2$ is not updated as well as when $X_2$ is also updated, and extending this to the whole fleet). This total cost function most likely has local minima, so when minimizing one should use a global optimization approach, such as cutting-plane methods or branch and bound methods, or reinforcement learning. S and t should be selected such that the total costs are minimal, and from that also follows which devices are updated simultaneously.

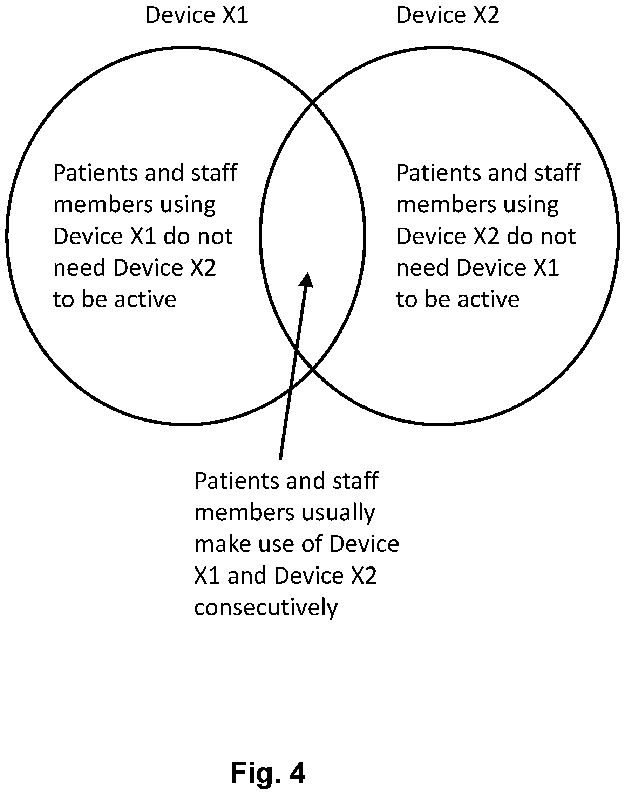

Fig. 4 shows a Venn diagram of determining whether to update or upgrade two devices **120** simultaneously or consecutively. To better consider the interdependency of a device $X_1$ interacting with device $X_2$ we propose to make use of the historical data of the hospital's databases to see how often device $X_1$ and device $X_2$ work together. This minimizes the risk of causing a major issue by having both devices down due to malfunctioning and/or unplanned maintenance tasks. For example, devices $X_1$ and $X_2$ can have a stronger interdependency link (intersection area) if, for example, certain procedures imply patients in the need of devices $X_1$ and $X_2$, staff members have the same competence to operate devices $X_1$ and $X_2$, and so forth. Different data sources such as procedure historical data (to see which devices are needed) or skills matrix data (staff competence vs devices he/she operates frequently). In any non-intersecting situation both devices $X_1$ and $X_2$ could be updated together (even in parallel) without creating major issues due to downtime.

The GUI **140** is configured to allow a user to visualize the impact of certain decisions. This way, users can simulate and try first, before making actual decisions and plan accordingly. In current practice, many people (from the hospital and from the provider) are involved in making this decision on which devices to update when. Moreover, the current scheduling systems don't take all the variables described and the interdependency issue. Such a GUI **140** can facilitate this communication and decision-making process.

The cost function under one scenario can be broken down into different elements of that scenario, i.e., costs of letting staff members work more or at other times, costs of delaying patients, etc. These costs should be set. To initialize the costs, for a device X, considerations can include staff members availability that normally operate device X will have higher associated costs than staff members that don't make use of such devices, patients which need due to their procedure make use of device X will have higher associated costs than patients which don't need such device., and so forth. To extract this information of need of device X by either staff members or patient historical data that the hospital held where the device X was in use can be used.

Afterwards, the system **100** can learn from the usage the planner does of the interactive tool. Per the sliders **142**, which is accessible to the planner through the GUI **140**, the model **130** can learn how much deviation is from the "suggested cost" by the system **100** and the "adjusted cost" that the planner adjusts for a device X. Through time this learning model **130** will become more accurate taking into consideration the degree of adjustment the planner normally does for a given device X. Other factors to consider in the cost initialization are, for example, some staff members are more flexibly deployable compared to others, for example because they have a broader set of skills and can also be allocated to other machines or tasks, some staff members might have a longer 'backlog' of tasks that they can do alternatively, when not operating, for example following e-learning courses, documentation tasks, or supervising colleagues, some staff members might deal easier with changes in their schedule than others, for example because they have less commute time to work, a more flexible situation at home, etc., some patients have more severe health issues than others and this should be prioritized to make use of the device to get updated, and so forth.

In some embodiments, a video can display the delta changes between the old and new updates and also show the ease at which the device can be used after the update. This feature can help the hospital to decide the downtime and time required during and after the update.

A non-transitory storage medium includes any medium for storing or transmitting information in a form readable by a machine (e.g., a computer). For instance, a machine-readable medium includes read only memory ("ROM"), solid state drive (SSD), flash memory, or other electronic storage medium; a hard disk drive, RAID array, or other magnetic disk storage media; an optical disk or other optical storage media; or so forth.

The methods illustrated throughout the specification, may be implemented as instructions stored on a non-transitory storage medium and read and executed by a computer or other electronic processor.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A system (**100**) for planning an upcoming update or upgrade of a fleet of medical devices (**120**), the system comprising

    a hardware processor (**101**, 113**);**
    a display device (**105**); and
    a non-transitory computer readable medium (**107**, **127**) storing instructions readable and executable by the hardware processor to:

        receive historical workload information for medical devices of a fleet of medical devices (**120**), the fleet including at least two medical devices;
        receive, via a user interface (UI) (**140**) presented on the display device, cost preferences for respective costs of the upcoming update or upgrade of the fleet of medical devices;

estimate values for the respective costs for different scenarios for performing the upcoming update or upgrade of the fleet of medical devices, each scenario specifying a schedule for the upcoming update or upgrade of the fleet of medical devices and resources allocated for the upcoming update or upgrade of the fleet of medical devices, wherein the values estimated for the costs for each scenario are metrics of the impact of performing the upcoming update or upgrade in accordance with the scenario on efficiency of operation of the fleet of medical devices during the upcoming update or upgrade;

select one or more proposed scenarios for performing the upcoming update or upgrade of the fleet of medical devices based on comparisons of the estimated values with the cost preferences for the respective costs received via the UI; and

display, on the display device, information about the selected one or more scenarios for performing the upcoming update or upgrade of the fleet of medical devices including at least indications of the estimated values for the respective costs.

2. The system (**100**) of claim 1, wherein the instructions further include:

receiving information on resources available for performing the upcoming update or upgrade of the fleet of medical devices (**120**);

wherein each scenario further specifies resources allocated for the upcoming update or upgrade of the fleet of medical devices.

3. The system (**100**) of either one of claims 1 and 2, wherein the different scenarios differ at least in whether the upcoming update or upgrade is performed simultaneously or consecutively for a pair of medical devices of the fleet of medical devices.

4. The system (**XX**) of any one of claims 1-3, wherein the UI (**140**) displays a slider bar (**142**) for each respective cost of the upcoming update or upgrade of the fleet of medical devices and the inputs setting the cost preferences are received as adjustments of the slider bars.

5. The system (**100**) of any one of claims 1-4, wherein the instructions are further readable and executable by the hardware processor to:

receive, via the UI (**140**), a selection of a user-selected scenario from the selected one or more scenarios; and

automatically perform at least a portion of the upcoming update or upgrade of the fleet of medical devices (**120**) in accordance with the user-selected scenario.

6. The system (**100**) of any one of claims 1-5, further storing a model (**130**) configured to analyze the cost preferences for respective costs of the upcoming update or upgrade of the fleet of medical devices to estimate the values for the respective costs for the different scenarios.

7. The system (**100**) of any one of claims 1-6, wherein each scenario further includes one or more of updates for the fleet of medical devices (**120**), patient care workflows, staff schedules, and field service engineer schedules.

8. A method (**200**) for planning an upcoming update or upgrade of a fleet of medical devices (**120**), the method comprising:

receiving historical workload information for medical devices of the fleet of medical devices (**120**);

receiving, via a user interface (UI) (**140**), cost preferences for respective costs of the upcoming update or upgrade of the fleet of medical devices;

estimating values for the respective costs of the upcoming update or upgrade of the fleet of medical devices for different scenarios for performing the upcoming update or upgrade of the fleet of medical devices, each scenario specifying at least a schedule for the upcoming update or upgrade of the fleet of medical devices and whether pairs of medical devices of the fleet are to be updated or upgraded simultaneously or consecutively;

selecting one or more proposed scenarios for performing the upcoming update or upgrade of the fleet of medical devices based on comparisons of the estimated values for the respective costs with the cost preferences for the respective costs received via the UI; and

outputting, on the UI, information about the selected one or more scenarios for performing the upcoming update or upgrade of the fleet of medical devices including at least indications of the estimated values for the respective costs.

9. The method (**100**) of claim 8, further comprising:

receiving information on resources available for performing the upcoming update or upgrade of the fleet of medical devices;
wherein each scenario further specifies resources allocated for the upcoming update or upgrade of the fleet of medical devices.

10. The method (**100**) of either one of claims 8 and 9, wherein the respective costs include at least a patient delay cost, a patient rescheduling cost, and a staff overtime cost, and the values estimated for the costs for each scenario are metrics of the impact of performing the upcoming update or upgrade in accordance with the scenario on efficiency of operation of the fleet of medical devices during the upcoming update or upgrade.

11. The method (**100**) of any one of claims 8-10, wherein the UI (**140**) displays a slider bar (**142**) for each respective cost of the upcoming update or upgrade of the fleet of medical devices and the inputs setting the cost preferences are received as adjustments of the slider bars.

12. The method (**100**) of any one of claims 8-11, further including:

determining default cost preferences based on cost preferences received via the UI (**140**) for previous updates or upgrades of the fleet of medical devices and/or similar fleets of medical devices; and
prior to receiving the cost preferences via the UI, initializing the UI with the default cost preferences.

13. The method (**100**) of any one of claims 8-12, further including:
with a model (**130**), analyzing the cost preferences for respective costs of the upcoming update or upgrade of the fleet of medical devices to estimate the values for the respective costs for the different scenarios.

14. The method (**100**) of any one of claims 8-13, further comprising:

receiving, via the UI (**140**), a selection of a user-selected scenario from the selected one or more scenarios; and
automatically performing at least a portion of the upcoming update or upgrade of the fleet of medical devices (**120**) in accordance with the user-selected scenario.

15. A non-transitory computer readable medium (**107, 127**) storing instructions readable and executable by at least one hardware processor (**101, 113**) to:

receive historical workload information for medical devices of a fleet of medical devices (**120**), the fleet including at least two medical devices;
receive information on an upcoming update or upgrade of the fleet of medical devices;
receive information on resources available for performing the upcoming update or upgrade of the fleet of medical devices;
receive, via a user interface (UI) (**140**), cost preferences for respective costs of the upcoming update or upgrade of the fleet of medical devices;
simulate different scenarios performing the upcoming update or upgrade of the fleet of medical devices, each scenario specifying a schedule for the upcoming update or upgrade of the fleet of medical devices and resources allocated for the upcoming update or upgrade of the fleet of medical devices, the simulation of each scenario utilizing the historical workload information for the medical devices of the fleet of medical devices and outputting estimated values for the respective costs of the upcoming update or upgrade of the fleet of medical devices;
select one or more proposed scenarios for performing the upcoming update or upgrade of the fleet of medical devices from the simulated different scenarios based on comparisons of the estimated values for the respective costs output by the simulations of the different scenarios with the cost preferences for the respective costs received via the UI; and
output, on the UI, information about the selected one or more scenarios for performing the upcoming update or upgrade of the fleet of medical devices including at least indications of the estimated values for the respective costs.

**Fig. 1**

200

```
┌─────────────────────────────┐
│   Receive historical info   │
│             202             │
└─────────────────────────────┘
              │
              ▼
┌─────────────────────────────┐
│   Receive inputs related to │
│ upgrade/update session 204  │
└─────────────────────────────┘
              │
              ▼
┌─────────────────────────────┐
│     Estimate costs based    │
│   on received inputs 206    │
└─────────────────────────────┘
              │
              ▼
┌─────────────────────────────┐
│   Select scenario(s) based  │
│   on estimated costs 208    │
└─────────────────────────────┘
              │
              ▼
┌─────────────────────────────┐
│      Display selected       │
│      scenario(s) 210        │
└─────────────────────────────┘
              │
              ▼
┌─────────────────────────────┐
│  Receive user selection of a│
│  user-selected scenario 212 │
└─────────────────────────────┘
              │
              ▼
┌─────────────────────────────┐
│  Perform update or upgrade  │
│    in accordance with the   │
│ user-selected scenario 214  │
└─────────────────────────────┘
```

**Fig. 2**

Fig. 3

EP 4 542 566 A1

Device X1           Device X2

Patients and staff
members using
Device X1 do not
need Device X2
to be active

Patients and staff
members using
Device X2 do not
need Device X1
to be active

Patients and staff
members usually
make use of Device
X1 and Device X2
consecutively

**Fig. 4**

Europäisches
Patentamt

European
Patent Office

Office européen
des brevets

# EUROPEAN SEARCH REPORT

Application Number

**EP 23 20 8229**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/046576 A1 (KONINKLIJKE PHILIPS NV [NL]) 30 March 2023 (2023-03-30) <br> * figures 1,3 * <br> * paragraphs [0026], [0027] * <br> * paragraphs [0039] – [0044] * <br> * paragraphs [0050] – [0055] * | 1-15 | INV. <br> G16H40/20 <br> G06Q10/06 |
| X | US 2021/134447 A1 (MINTZ ILAN [US]) 6 May 2021 (2021-05-06) <br> * paragraphs [0033] – [0040] * <br> * paragraphs [0049] – [0059] * <br> * paragraphs [0072], [0080] * | 1-15 | |
| X | US 2021/056575 A1 (MINTZ ILAN [US]) 25 February 2021 (2021-02-25) <br> * paragraphs [0007] – [0009], [0027] – [0029], [0040]; figures 1-12 * | 1-15 | |
| A | US 2022/051184 A1 (CELLA CHARLES HOWARD [US] ET AL) 17 February 2022 (2022-02-17) <br> * the whole document * | 1-15 | |
| A | GURBETA LEJLA ET AL: "Inspection process of medical devices in healthcare institutions: software solution", HEALTH AND TECHNOLOGY, SPRINGER BERLIN HEIDELBERG, BERLIN/HEIDELBERG, vol. 7, no. 1, 7 December 2016 (2016-12-07), pages 109-117, XP036179239, ISSN: 2190-7188, DOI: 10.1007/S12553-016-0154-2 [retrieved on 2016-12-07] <br> * the whole document * | 1-15 | TECHNICAL FIELDS SEARCHED (IPC) <br><br> G16H <br> G06Q |

The present search report has been drawn up for all claims

| Place of search | Date of completion of the search | Examiner |
|---|---|---|
| Munich | 17 April 2024 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 20 8229**

This annex lists the patent family members relating to the patent documents cited in the above-mentioned European search report.
The members are as contained in the European Patent Office EDP file on
The European Patent Office is in no way liable for these particulars which are merely given for the purpose of information.

**17-04-2024**

| Patent document cited in search report | Publication date | Patent family member(s) | | Publication date |
|---|---|---|---|---|
| WO 2023046576 A1 | 30-03-2023 | NONE | | |
| US 2021134447 A1 | 06-05-2021 | NONE | | |
| US 2021056575 A1 | 25-02-2021 | NONE | | |
| US 2022051184 A1 | 17-02-2022 | AU | 2020379834 A1 | 09-06-2022 |
| | | CN | 115699050 A | 03-02-2023 |
| | | JP | 2023500378 A | 05-01-2023 |
| | | US | 2022035341 A1 | 03-02-2022 |
| | | US | 2022035342 A1 | 03-02-2022 |
| | | US | 2022036274 A1 | 03-02-2022 |
| | | US | 2022036275 A1 | 03-02-2022 |
| | | US | 2022036276 A1 | 03-02-2022 |
| | | US | 2022036301 A1 | 03-02-2022 |
| | | US | 2022036302 A1 | 03-02-2022 |
| | | US | 2022044204 A1 | 10-02-2022 |
| | | US | 2022051171 A1 | 17-02-2022 |
| | | US | 2022051184 A1 | 17-02-2022 |
| | | US | 2022051361 A1 | 17-02-2022 |
| | | US | 2022058569 A1 | 24-02-2022 |

EPO FORM P0459

For more details about this annex : see Official Journal of the European Patent Office, No. 12/82